# EUROPEAN PATENT APPLICATION

(11) **EP 4 748 316 A2**
(43) Date of publication of application: **27.05.2026**
(21) Application number: 26151315.4
(22) Date of filing: 10.02.2021
(51) Int. Cl.: A61B 8/00

(54) **IMAGING MEDICAL DEVICE SYSTEMS WITH A BUBBLE-REDUCING MEMBER**

(30) Priority: 12.02.2020 US 202062975517 P
(62) Divisional of application: 25151088.9
(71) Applicant: Boston Scientific Scimed, Inc., Maple Grove, Minnesota 55311 (US)
(72) Inventor: PLUNGER, Bryan Joseph, Minneapolis, 55411 (US); IMURA, Haruka, Minneapolis, 55403 (US); FLEURY, Sean P., Princeton, 01541 (US); FULLER, Jeffrey Steven, Brooklyn Park, 55444 (US); ORTIZ SERRANO, Monica, Maple Groove, 55311 (US); SEPPALA, Jan, Loretto, 55357 (US); HAMMERSTROM, Jeffrey Adam, New Hope, 55427 (US); SZLAG, Victoria, St. Paul, 55108 (US)
(74) Representative: Vossius & Partner Patentanwälte Rechtsanwälte mbB

(57) **Abstract**

The present invention relates to an imaging medical device, comprising an elongate shaft having a distal end region, an imaging assembly disposed within the elongate shaft, the imaging assembly including a drive cable, a housing coupled to the drive cable, and a transducer coupled to the drive cable and/or the housing, and a bubble-reducing member designed so that when the imaging medical device is flushed, the bubble-reducing member disrupts bubbles travelling toward the housing and transducer.

## Description

### Cross-Reference To Related Applications

This application claims the benefit of priority under 35 U.S.C. §119 to U.S. Provisional Application Serial No. 62/975,517, filed February 12, 2020, the entirety of which is incorporated herein by reference.

### Technical Field

The present disclosure pertains to medical devices, and methods for manufacturing medical devices. More particularly, the present disclosure pertains to medical devices for imaging.

### Background

A wide variety of intracorporeal medical devices have been developed for medical use, for example, intravascular use. Some of these devices include guidewires, catheters, and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and using medical devices.

### Brief Summary

This disclosure provides design, material, manufacturing method, and use alternatives for medical devices. An imaging medical device is disclosed. The imaging medical device comprises: an elongate shaft having a distal end region; an imaging assembly disposed within the elongate shaft, the imaging assembly including a drive cable, a housing coupled to the drive cable, and a transducer coupled to the housing; and a bubble-reducing member disposed adjacent to the drive cable.

Alternatively or additionally to any of the embodiments above, the imaging assembly is rotatable within the elongate shaft.

Alternatively or additionally to any of the embodiments above, the imaging assembly is translatable within the elongate shaft.

Alternatively or additionally to any of the embodiments above, the bubble-reducing member is coupled to the drive cable.

Alternatively or additionally to any of the embodiments above, the bubble-reducing member extends between the drive cable and an inner surface of the elongate shaft.

Alternatively or additionally to any of the embodiments above, the bubble-reducing member includes a barrier disk with a plurality of openings formed therein.

Alternatively or additionally to any of the embodiments above, the bubble-reducing member includes a tapered mesh.

Alternatively or additionally to any of the embodiments above, the bubble-reducing member includes a plurality of axially-extending fingers.

Alternatively or additionally to any of the embodiments above, the bubble-reducing member includes a region with a surface treatment.

Alternatively or additionally to any of the embodiments above, the bubble-reducing member includes a coating.

Alternatively or additionally to any of the embodiments above, the transducer includes an ultrasound transducer.

An imaging medical device is disclosed. The imaging medical device comprises: a catheter having a proximal end region and a distal end region; an imaging assembly movably disposed within the catheter, the imaging assembly including a drive cable, a housing coupled to the drive cable, and an ultrasound transducer coupled to the housing; and a bubble-reducing member coupled to the drive cable, the bubble-reducing member being configured to allow fluid to flow between the proximal end region to the distal end region while disrupting the flow of bubbles between the proximal end region and the distal end region.

Alternatively or additionally to any of the embodiments above, the imaging assembly is rotatable within the catheter.

Alternatively or additionally to any of the embodiments above, the imaging assembly is axially translatable within the catheter.

Alternatively or additionally to any of the embodiments above, the bubble-reducing member extends between the drive cable and an inner surface of the catheter.

Alternatively or additionally to any of the embodiments above, the bubble-reducing member includes a barrier disk with a plurality of openings formed therein.

Alternatively or additionally to any of the embodiments above, the bubble-reducing member includes a tapered mesh.

Alternatively or additionally to any of the embodiments above, the bubble-reducing member includes a plurality of axially-extending fingers.

Alternatively or additionally to any of the embodiments above, the bubble-reducing member includes a hydrophobic region.

An imaging medical device is disclosed. The imaging medical device comprises: a catheter having a proximal end region and a distal end region; an imaging assembly disposed within the catheter, the imaging assembly being rotatable and translatable relative to the catheter and including a drive cable, a housing coupled to the drive cable, and an ultrasound transducer coupled to the housing; and a bubble-reducing member coupled to the drive cable, the bubble-reducing member being configured to allow fluid to flow between the proximal end region to the distal end region while reducing the flow of bubbles between the proximal end region and the distal end region.

The above summary of some embodiments is not intended to describe each disclosed embodiment or every implementation of the present disclosure. The Figures, and Detailed Description, which follow, more particularly exemplify these embodiments.

### Brief Description of the Drawings

The disclosure may be more completely understood in consideration of the following detailed description in connection with the accompanying drawings, in which:
FIG. 1 is a side view of an example medical device.
FIG. 2 is a side view of a portion of an example medical device.
FIG. 3 is a partial cross-sectional view of a portion of an example medical device.
FIG. 4 is a partial cross-sectional view of a portion of an example medical device.
FIG. 5 is a partial cross-sectional view of a portion of an example medical device.
FIG. 6 is a partial cross-sectional view of a portion of an example medical device.

While the disclosure is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the spirit and scope of the disclosure.

### Detailed Description

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the invention.

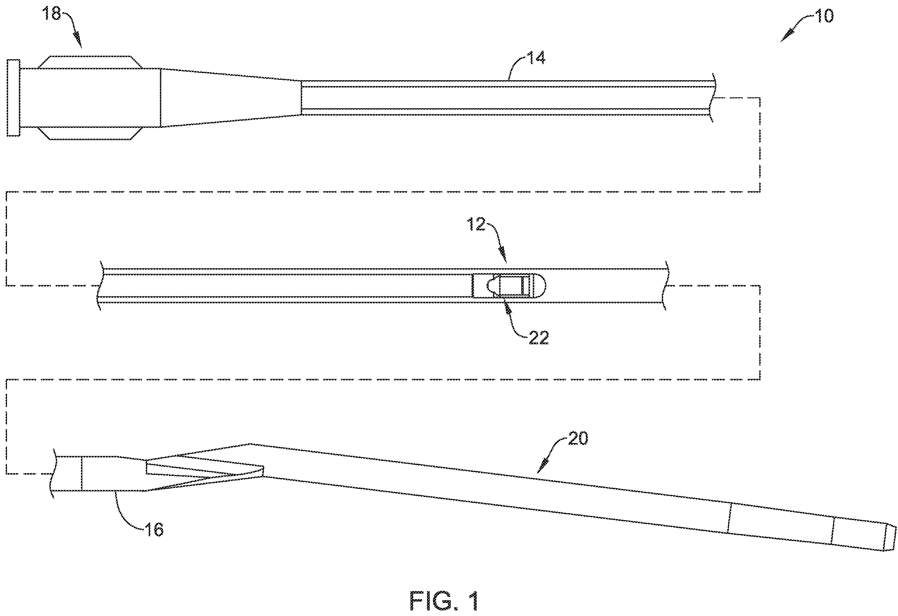

FIG. 1 is a side view of an example medical device 10. In at least some instances, the medical device 10 takes the form of an imaging medical device. For example, the medical device 10 may be an intravascular ultrasound (IVUS) device that may be used to image a blood vessel. The structure/form of the medical device 10 can vary. In some instances, the medical device 10 may include an elongate shaft 12 having a proximal end region 14 and a distal end region 16. A hub 18 may be coupled to or otherwise disposed adjacent to the proximal end region 14. A tip member 20 may be coupled to or otherwise disposed adjacent to the distal end region 16. The tip member 20 may include a guidewire lumen, an atraumatic distal end, one or more radiopaque markers, and/or other features. An imaging assembly 22 may be disposed within the shaft 12. In general, the imaging assembly may be used to capture/generate images of a blood vessel. In some instances, the medical device may include devices and/or features similar to those disclosed in U.S. Patent Application Pub. No. US 2012/0059241 and U.S. Patent Application Pub. No. US 2017/0164925, the entire disclosures of which are herein incorporated by reference. In at least some instances, the medical device 10 may resemble and/or include features that resemble the OPTICROSS^{™} Imaging Catheter, commercially available from BOSTON SCIENTIFIC, Marlborough, MA.

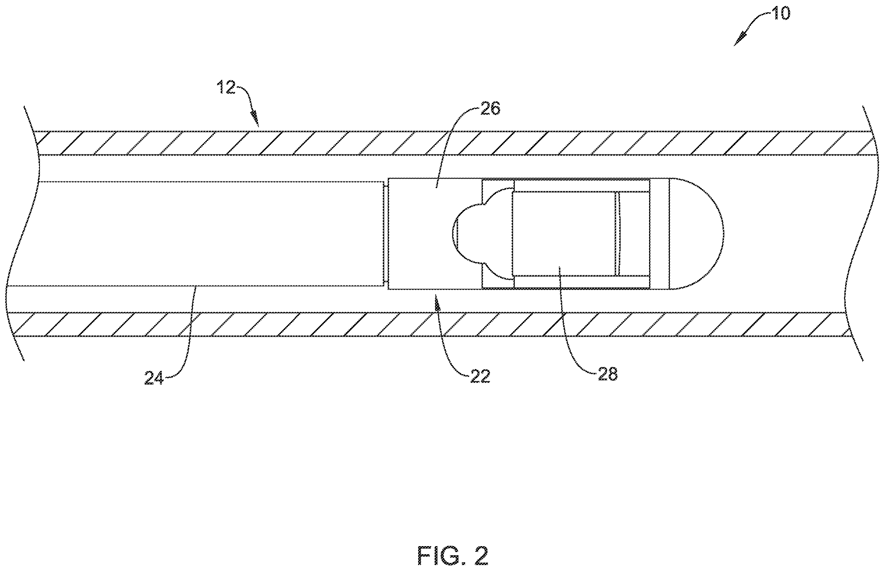

The imaging assembly 22 may include a drive cable or shaft 24, a housing 26, and an imaging member or transducer 28 coupled to the drive cable 24 and/or housing 26 as shown in FIG. 2. In at least some instances, the transducer 28 includes an ultrasound transducer. Other transducers are also contemplated. The transducer 28 may be rotatable and/or axially translatable relative to the shaft 12. For example, the drive cable 24 may be rotated and/or translated in order to rotate and/or translate the transducer 28 (and the housing 26).

When using the medical device 10, it may be desirable to prepare and/or flush the shaft 12. In order to flush the medical device 10, fluid may be infused at a flush port on or at the hub 18. The fluid may exit the medical device at a vent hole (not shown) adjacent to the distal end of the housing 26. In some instances, the flushing process may result in the formation of bubbles within the shaft 12. It may be desirable to flush the medical device 10 in a manner that reduces the formation of bubbles and/or removes/disrupts any bubbles that are formed because bubbles may reflect/disrupt a signal (e.g., an ultrasound signal) from the transducer 28, which disrupts the image. While flushing is generally effective for removing bubbles, some bubbles may still get caught within the shaft 12. Disclosed herein are medical devices that are designed to help reduce the formation of bubbles and/or that are designed to help disrupt bubbles that may be formed within the medical device.

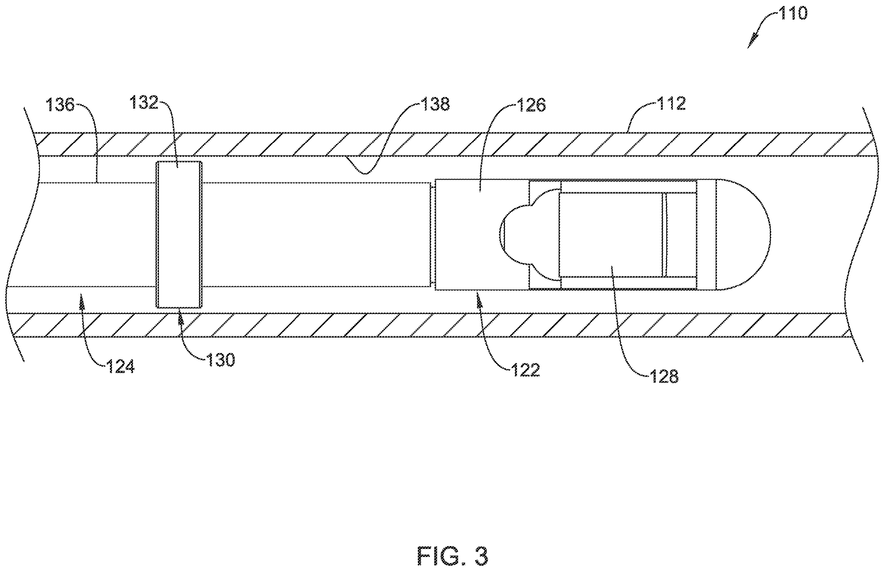

FIG. 3 illustrates a portion of another example medical device 110 that may be similar in form and function to other medical devices disclosed herein. The medical device 110 may include an elongate shaft 112. An imaging assembly 122 may be disposed within the shaft 112. The imaging assembly 122 may include a drive cable or shaft 124, a housing 126, and an imaging member or transducer 128 coupled to the drive cable 124 and/or housing 126.

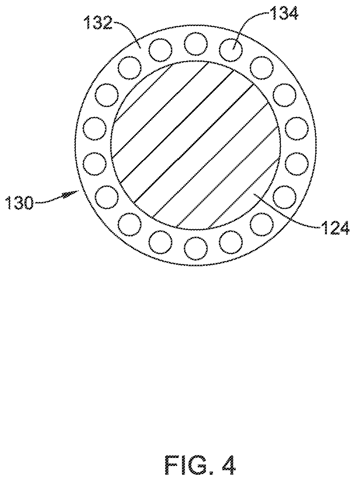

A bubble-reducing member 130 may be coupled to or otherwise disposed adjacent to the drive cable 124. The bubble-reducing member 130 may include a barrier or annular portion 132. A plurality of apertures 134 may be disposed within the barrier portion 132 as can be seen in FIG. 4. In FIG. 4, the drive cable 124 is depicted schematically with cross-hatch. In general, the bubble-reducing member 130 is designed so that when the medical device 110 is flushed, the bubble-reducing member 130 blocks or reduces bubbles from travelling along the shaft 112 toward the housing 126 and transducer 128. More particularly, the barrier portion 132 serves as a barrier to the flow of bubbles. In at least some instances, the bubble-reducing member 130 (e.g., the barrier portion 132) extends between the outer surface 136 of the drive cable 124 and an inner surface 138 of the elongate shaft 112. This may include the barrier portion 132 contacting the inner surface 138 of the shaft 112 or being disposed adjacent to the inner surface 138 of the shaft 112.

The apertures 134 are designed to allow for fluid to pass therethrough. For example, the apertures 134 may be sized so that fluid can pass therethrough while bubbles are either prevented from passing therethrough or are disrupted/broken when passing therethrough in manner that reduces the impact of bubble on the generation of images. In other words, larger bubbles are substantially prevented from passing through the apertures 134 and only smaller bubbles (e.g., small enough so that they can readily escape the vent hole) can pass therethrough. For example, the apertures may have a diameter of about 0.001-0.01 inches, or about 0.003-0.005 inches. Bubbles passing through such apertures would be unlikely to adhere to the transducer 128 and/or otherwise be unlikely to impact the transducer 128.

In some instances, the bubble-reducing member 130 may include a surface treatment and/or other structural feature that also helps to reduce bubbles. For example, the bubble-reducing member 130 may be surface treated, etched (e.g., chemically etched), treated/coated with a coating such as polytetrafluoroethylene, micropatterned, coated (e.g., including a coating), or otherwise made to be hydrophobic (e.g., super-hydrophobic). This may cause any bubbles formed to preferentially stick to the surface treated region rather than migrating toward the transducer 128.

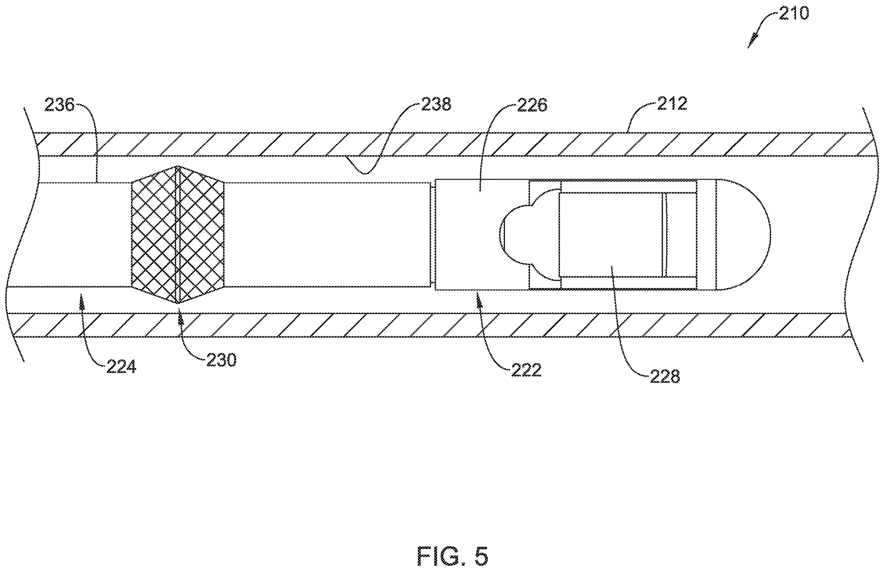

FIG. 5 illustrates a portion of another example medical device 210 that may be similar in form and function to other medical devices disclosed herein. The medical device 210 may include an elongate shaft 212. An imaging assembly 222 may be disposed within the shaft 212. The imaging assembly 222 may include a drive cable or shaft 224, a housing 226, and an imaging member or transducer 228 coupled to the drive cable 224 and/or housing 226.

A bubble-reducing member 230 may be coupled to or otherwise disposed adjacent to the drive cable 224. The bubble-reducing member 230 may take the form of a tapered mesh. The bubble-reducing member/tapered mesh 230 may function similarly to the bubble-reducing member 130. For example, the bubble-reducing member 230 may extend between the outer surface 236 of the drive cable 224 and an inner surface 238 of the elongate shaft 212 so as to form a barrier while the mesh-like structure (e.g., with openings therein) allows for fluid to pass therethrough (while substantially preventing bubbles from passing therethrough).

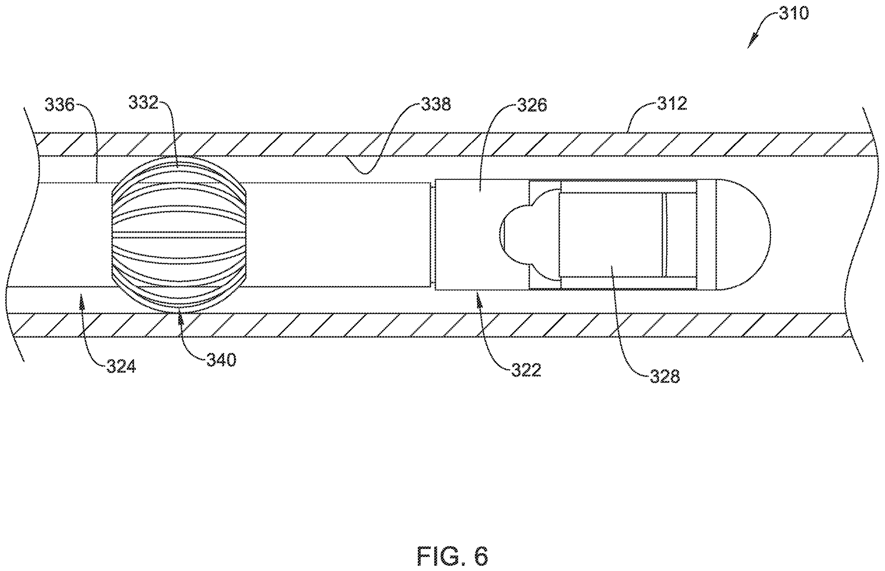

FIG. 6 illustrates a portion of another example medical device 310 that may be similar in form and function to other medical devices disclosed herein. The medical device 310 may include an elongate shaft 312. An imaging assembly 322 may be disposed within the shaft 312. The imaging assembly 322 may include a drive cable or shaft 324, a housing 326, and an imaging member or transducer 328 coupled to the drive cable 324 and/or housing 326.

A bubble-reducing member 330 may be coupled to or otherwise disposed adjacent to the drive cable 324. The bubble-reducing member 330 may include a plurality of axially-extending fingers 340. The bubble-reducing member 330 may function similarly to the bubble-reducing members 130/230. For example, in at least some instances, the barrier member 330 extends between the outer surface 336 of the drive cable 324 and an inner surface 338 of the elongate shaft 312 so as to form a barrier. The spaces between the axially-extending fingers 340 allow for fluid to pass therethrough (while substantially preventing bubbles from passing therethrough).

The materials that can be used for the various components of the medical device 10 and/or other medical devices are disclosed herein. For simplicity purposes, the following discussion makes reference to the medical device 10. However, this is not intended to limit the devices and methods described herein, as the discussion may be applied to other similar tubular members and/or components of tubular members or devices disclosed herein.

The medical device 10 may be made from a metal, metal alloy, polymer (some examples of which are disclosed below), a metal-polymer composite, ceramics, combinations thereof, and the like, or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), high-density polyethylene (e.g., MARLEX^{®} high-density polyethylene), low-density polyethylene (e.g., MARLEX^{®} low-density polyethylene), linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R30035 such as MP35-N^{®} and the like), nickel-molybdenum alloys (e.g., UNS: N10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY^{®}, PHYNOX^{®}, and the like); platinum enriched stainless steel; titanium; combinations thereof; and the like; or any other suitable material.

In at least some embodiments, portions or all of the medical device 10 may also be doped with, made of, or otherwise include a radiopaque material. Radiopaque materials are understood to be materials capable of producing a relatively bright image on a fluoroscopy screen or another imaging technique during a medical procedure. This relatively bright image aids the user of the medical device 10 in determining its location. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of the medical device 10 to achieve the same result.

In some embodiments, a degree of Magnetic Resonance Imaging (MRI) compatibility is imparted into the medical device 10. For example, the medical device 10, or portions thereof, may be made of a material that does not substantially distort the image and create substantial artifacts (e.g., gaps in the image). Certain ferromagnetic materials, for example, may not be suitable because they may create artifacts in an MRI image. The medical device 10, or portions thereof, may also be made from a material that the MRI machine can image. Some materials that exhibit these characteristics include, for example, tungsten, cobalt-chromium-molybdenum alloys (e.g., UNS: R30003 such as ELGILOY^{®}, PHYNOX^{®}, and the like), nickel-cobalt-chromium-molybdenum alloys (e.g., UNS: R30035 such as MP35-N^{®} and the like), nitinol, and the like, and others.

U.S. Patent Application Pub. No. US 2012/0059241 is herein incorporated by reference.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The invention's scope is, of course, defined in the language in which the appended claims are expressed.
The following aspects are preferred embodiments of the present invention.
1. An imaging medical device, comprising:
   an elongate shaft having a distal end region;
   an imaging assembly disposed within the elongate shaft, the imaging assembly including a drive cable, a housing coupled to the drive cable, and a transducer coupled to the housing; and
   a bubble-reducing member disposed adjacent to the drive cable.
2. The imaging medical device of aspect 1, wherein the imaging assembly is rotatable within the elongate shaft.
3. The imaging medical device of any one of aspects 1-2, wherein the imaging assembly is translatable within the elongate shaft.
4. The imaging medical device of any one of aspects 1-3, wherein the bubble-reducing member is coupled to the drive cable.
5. The imaging medical device of any one of aspects 1-4, wherein the bubble-reducing member extends between the drive cable and an inner surface of the elongate shaft.
6. The imaging medical device of any one of aspects 1-5, wherein the bubble-reducing member includes a barrier disk with a plurality of openings formed therein.
7. The imaging medical device of any one of aspects 1-5, wherein the bubble-reducing member includes a tapered mesh.
8. The imaging medical device of any one of aspects 1-5, wherein the bubble-reducing member includes a plurality of axially-extending fingers.
9. The imaging medical device of any one of aspects 1-8, wherein the bubble-reducing member includes a region with a surface treatment.
10. The imaging medical device of any one of aspects 1-9, wherein the bubble-reducing member includes a coating.
11. The imaging medical device of any one of aspects 1-10, wherein the transducer includes an ultrasound transducer.
12. An imaging medical device, comprising:
   a catheter having a proximal end region and a distal end region;
   an imaging assembly movably disposed within the catheter, the imaging assembly including a drive cable, a housing coupled to the drive cable, and an ultrasound transducer coupled to the housing; and
   a bubble-reducing member coupled to the drive cable, the bubble-reducing member being configured to allow fluid to flow between the proximal end region to the distal end region while disrupting the flow of bubbles between the proximal end region and the distal end region.
13. The imaging medical device of aspect 12, wherein the bubble-reducing member includes a barrier disk with a plurality of openings formed therein.
14. The imaging medical device of aspect 12, wherein the bubble-reducing member includes a tapered mesh.
15. The imaging medical device of aspect 12, wherein the bubble-reducing member includes a plurality of axially-extending fingers.

## Claims

1. An imaging medical device, comprising:
an elongate shaft having a distal end region;
an imaging assembly disposed within the elongate shaft, the imaging assembly including a drive cable, a housing coupled to the drive cable, and a transducer coupled to the drive cable and/or the housing; and
a bubble-reducing member designed so that when the imaging medical device is flushed, the bubble-reducing member disrupts bubbles travelling toward the housing and transducer.

2. The imaging medical device of claim 1, wherein the bubble-reducing member disrupts bubbles travelling along the elongate shaft toward the housing and transducer.

3. The imaging medical device of claim 1 or 2, wherein the bubble-reducing member includes a barrier or annular portion with a plurality of apertures disposed therein.

4. The imaging medical device of claim 1 or 2, wherein the bubble-reducing member includes a tapered mesh.

5. The imaging medical device of claim 1 or 2, wherein the bubble-reducing member includes a plurality of axially-extending fingers.

6. The imaging medical device of any one of claims 1-5, wherein the imaging assembly is rotatable and/or translatable within the elongate shaft.

7. The imaging medical device of any one of claims 1-6, wherein the bubble-reducing member is coupled to the drive cable.

8. The imaging medical device of any one of claims 1-7, wherein the bubble-reducing member extends between the drive cable and an inner surface of the elongate shaft.

9. The imaging medical device of any one of claims 1-8, wherein the bubble-reducing member includes a region with a surface treatment.

10. The imaging medical device of any one of claims 1-9, wherein the bubble-reducing member includes a coating.

11. The imaging medical device of any one of claims 1-12, wherein the transducer includes an ultrasound transducer.

12. The imaging medical device of any one of claims 1-11, wherein the elongate shaft further has a proximal end region,
wherein the bubble-reducing member is coupled to the drive cable and configured to allow fluid to flow between the proximal end region to the distal end region while disrupting the flow of bubbles between the proximal end region and the distal end region, and
wherein the imaging assembly is movably disposed within the elongate shaft.

13. The imaging medical device of any one of claims 1-12, wherein the bubble-reducing member is configured to block bubbles from passing.

14. The imaging device of any one of claims 1-13, wherein the bubble-reducing member is designed so that when the imaging medical device is flushed, fluid can pass therethrough while the bubbles are disrupted when passing therethrough.

15. The imaging device of any one of claims 1-14, wherein, when the imaging medical device is flushed, the bubble-reducing member blocks or reduces bubbles in order to disrupt bubbles travelling toward the housing and transducer.
